Europäisches Patentamt

European Patent Office

Office européen des brevets

(19)

(11) Veröffentlichungsnummer: **0 241 889**
**A2**

(12) # EUROPÄISCHE PATENTANMELDUNG

(21) Anmeldenummer: 87105415.1

(22) Anmeldetag: 11.04.87

(51) Int. Cl.4: **A61K 37/02** , A61K 37/24

(30) Priorität: 18.04.86 DE 3613167

(43) Veröffentlichungstag der Anmeldung:
**21.10.87 Patentblatt 87/43**

(84) Benannte Vertragsstaaten:
**AT BE CH DE ES FR GB IT LI NL SE**

(71) Anmelder: **BASF Aktiengesellschaft
Carl-Bosch-Strasse 38
D-6700 Ludwigshafen(DE)**

(72) Erfinder: **Urbaschek, Renate, Dr.
Trübnerstrasse 39
D-6900 Heidelberg(DE)**
Erfinder: **Urbaschek, Bernhard, Prof. Dr.
Trübnerstrasse 39
D-6900 Heidelberg(DE)**
Erfinder: **Maennel, Daniela, Dr.
Am Kirchweg 2
D-6901 Gaiberg(DE)**

(54) **Verwendung von TNF zur Herstellung von Arzneimitteln.**

(57) Es wird die Verwendung von TNF zur Prophylaxe und Therapie von Strahlenschäden beschrieben.

EP 0 241 889 A2

# Verwendung von TNF zur Herstellung von Arzneimitteln

TNF (Tumornekrosefaktor) ist eine Tumor-zerstörende Substanz, die zur Bekämpfung maligner Tumoren Verwendung finden soll. Die Struktur dieser Substanz ist in Natur 312 , 724 (1984) beschrieben worden.

Es wurde nun gefunden, daß sich TNF auch zur Prophylaxe und Therapie von Strahlenschäden eignet.

Gegenstand der Erfindung ist die Verwendung von TNF zur Herstellung von Arzneimitteln zur Prophylaxe und Therapie von Strahlenschäden bzw. die Verwendung von TNF zur Prophylaxe und Therapie von Strahlenschäden.

Strahlenschäden machen sich beispielsweise durch Knochenmarksuppression, Infektionskrankheiten, gastrointestinale Störungen und/oder zentralnervöse Schäden bemerkbar.

Da der TNF ein Polypeptid darstellt, welches im Magen-Darm-Trakt zerstört wird, kann er nur parenteral, vorzugsweise intavenös, verabfolgt werden. Hierzu eignen sich sterile isotonische Lösungen. Diese lassen sich beispielsweise herstellen, indem man den TNF in einer blutisotonischen wäßrigen Lösung löst, die Lösung steril filtriert und in Ampullen abfüllt. Der pH-Wert der Lösung liegt vorzugsweise zwischen 5 und 8, insbesondere bei etwa 7,5.

Die zu applizierende Dosis liegt bei 0,1 bis 5 mg, vorzugsweise 0,5 bis 3 mg TNF pro Patient und Tag. Die Behandlungsdauer beträgt in der Regel 1 bis 6 Tage.

Die Wirksamkeit des TNF ließ sich beispielsweise wie folgt zeigen:

Je 40 C3H/HEJ-Mäusen wurden einmal mit 660 rad Röntgenstrahlen bestraht. Je 20 Mäusen war 24 h vor der Bestrahlung und je 20 Mäusen 24 h nach der Bestrahlung 5 $\mu$g TNF i.v. injiziert worden. Die Mäuse wurden über einen Zeitraum von 30 Tagen nach der Bestrahlung auf Letalität beobachtet. Innerhalb dieses Zeitraums starben 40 % der Tiere. Von einer nicht mit TNF behandelten Kontrollgruppe starben unter den gleichen Bedingungen 75 % der Tiere.

## Herstellung einer Applikationsform

100 mg TNF werden in 300 ml 20 mM Natriumphosphatpuffer ph 7,5 gelöst. Die Lösung wird mit Kochsalz blutisotonisch eingestellt, über einen Porenfilter (Porengröße 0,1 bis 0,2 $\mu$) steril filtriert und jeweils 5 ml steril in Ampullen gefüllt.

## Ansprüche

Verwendung von TNF zur Herstellung von Arzneimitteln zur Prophylaxe und Therapie von Strahlenschäden.